# EUROPEAN PATENT APPLICATION

(11) **EP 3 699 258 A1**
(43) Date of publication of application: **26.08.2020**
(21) Application number: 18868290.0
(22) Date of filing: 10.10.2018
(51) Int. Cl.: C10L 1/02, B01J 21/04, B01J 21/06, B01J 23/30, C07C 67/03, C07C 69/24, C07C 69/52, C11C 1/00, C11C 3/10

(54) **METHOD FOR MANUFACTURING BIO-LIQUID FUEL**

(30) Priority: 16.10.2017 JP 2017200111
(71) Applicant: Nippon Soda Co., Ltd., Tokyo 100-8165 (JP)
(72) Inventor: KOMIYAMA Masaharu, Kofu-shi Yamanashi 400-0074 (JP)
(74) Representative: Wibbelmann, Jobst
(86) International application number: PCT/JP2018/037727
(87) International publication number: WO 2019/078061

(57) **Abstract**

A method for manufacturing a bio-liquid fuel wherein microalgae containing an oil/fat are brought into contact with a supercritical or subcritical methanol, or a supercritical or subcritical ethanol, in the presence of a catalyst that is an oxide containing at least one metal selected from the group consisting of metals of group 2 to group 13 in the periodic table, lanthanoids and actinoids.

## Description

### [Technical Field]

The present invention relates to a method for manufacturing bio-liquid fuel.

Priority is claimed on Japanese Patent Application No. 2017-200111, filed October 16, 2017, the entire disclosure of which is incorporated herein by reference.

### [Background Art]

The movement towards using aliphatic methyl esters obtained by reactions between vegetable oils and methanol as bio-liquid fuels is spreading. As a method for manufacturing aliphatic methyl esters from a vegetable oil, a method of using an alkaline catalyst such as NaOH or KOH in addition to the vegetable oil and methanol in order to make a transesterification reaction progress is well known. However, this alkaline catalyst method had a problem in that the alkaline catalyst could react with free fatty acid components contained in oils and fats, thereby creating soap as a byproduct. In order to solve this problem, Patent Document 1 proposes inducing a reaction with a vegetable oil under conditions in which the alcohol is in a supercritical state.

Additionally, in recent years, research and development has progressed towards using oils contained in microalgae as bio-liquid fuels. For example, Patent Document 2 proposes a method for generating fatty acid esters, including generating fatty acid esters by reacting microalgae with alcohol in a supercritical state. In this Patent Document 2, the use of a catalyst in the reaction between the microalgae and the alcohol is mentioned, but the problems solved thereby, and the functions and effects when using a catalyst, are not specifically disclosed, and no examples using catalysts are disclosed. In the invention in Patent Document 2, particularly when the reactions were performed in a state in which the microalgae were not dried and thus contained water, as indicated in Examples 1 and 2, there was a problem in that a very large quantity of solids remained after the reaction, thus requiring labor to separate and purify the oils.

### [Related Literature]

### [Patent Literature]

[Patent Document 1] JP 2000-109883 A
[Patent Document 2] JP 2010-503703 A

### [Summary of Invention]

### [Technical Problem]

The present invention addresses the problem of providing a method for manufacturing a bio-liquid fuel, wherein cell wall destruction, oil extraction and esterification are simultaneously and efficiently performed in a single step.

### [Solution to Problem]

In order to solve the above-mentioned problem, the present invention employs the features indicated below.
(1) A method for manufacturing a bio-liquid fuel wherein microalgae containing an oil/fat are brought into contact with a supercritical or subcritical methanol, or a supercritical or subcritical ethanol, in the presence of a catalyst that is an oxide containing at least one metal selected from the group consisting of metals of group 2 to group 13 in the periodic table, lanthanoids and actinoids.
(2) The method for manufacturing a bio-liquid fuel as in (1), wherein the catalyst is an oxide containing a transition metal.
(3) The method for manufacturing a bio-liquid fuel as in (1) or (2), wherein the catalyst is a catalyst supported on a carbon material or an aluminum oxide.
(4) The method for manufacturing a bio-liquid fuel as in any one of (1) to (3), wherein the catalyst is a composite oxide containing a transition metal.
(5) The method for manufacturing a bio-liquid fuel as in any one of (1) to (4), wherein the catalyst is a composite oxide containing zirconium.
(6) The method for manufacturing a bio-liquid fuel as in any one of (1) to (5), wherein the catalyst is a composite oxide containing zirconium and tungsten.
(7) The method for manufacturing a bio-liquid fuel as in any one of (1) to (6), wherein the catalyst is zirconia tungstate.
(8) The method for manufacturing a bio-liquid fuel as in any one of (1) to (7), wherein at least one of the microalgae containing the oil/fat, the supercritical or subcritical methanol, and the supercritical or subcritical ethanol contains water.
(9) The method for manufacturing a bio-liquid fuel as in any one of (1) to (8), wherein the microalgae containing the oil/fat are brought into contact with the supercritical or subcritical methanol, or the supercritical or subcritical ethanol, at a temperature from ordinary temperature to 400 °C and at a pressure from ordinary pressure to 50 MPa.
(10) The method for manufacturing a bio-liquid fuel as in any one of (1) to (9), wherein the microalgae containing the oil/fat are brought into contact with the supercritical or subcritical methanol, or the supercritical or subcritical ethanol, at a temperature from 200 to 400 °C and at a pressure from ordinary pressure to 50 MPa.

### [Advantageous Effects of Invention]

According to the method for manufacturing a bio-liquid fuel in the present invention, a step of destroying the cell walls of microalgae, a step of converting triglycerides contained in the microalgae to fatty acid esters, and a step of extracting the generated fatty acid esters can be performed simultaneously and efficiently, in a single step. Additionally, oils other than triglycerides contained in the microalgae can also be extracted. Furthermore, some of the organic matter other than oils contained in the microalgae can be converted to oils and extracted, thereby allowing the extraction of more oils than the amount that was stored as oils in the microalgae.

Therefore, the method for manufacturing the bio-liquid fuel in the present invention is an excellent method for extracting oils such as fatty acid esters from microalgae.

### [Brief Description of Drawings]

Fig. 1 is a graph indicating the temperature dependence of the carbon yield from oils and solids when dried *Chlorella* is reacted with anhydrous methanol in Comparative Example 1.
Fig. 2 is a graph indicating the effects of catalysts on the carbon yields when dried *Chlorella* is reacted with anhydrous and 20% water-containing methanol at 385 °C in Comparative Examples 1 and 2, and Examples 1 to 6.
Fig. 3 is a graph indicating fatty acid ester yields from oils obtained from dried *Chlorella* in Comparative Example 1.
Fig. 4 is a graph indicating fatty acid ester yields from oils obtained from dried *Chlorella* in Comparative Examples 1 and 2, and Examples 1 to 6.

### [Description of Embodiments]

Hereinafter, an embodiment of the method for manufacturing a bio-liquid fuel according to the present invention will be explained.

The method for manufacturing the bio-liquid fuel according to an embodiment of the present invention is a method for manufacturing a bio-liquid fuel wherein microalgae containing an oil/fat are brought into contact with a supercritical or subcritical methanol, or a supercritical or subcritical ethanol, in the presence of a specific catalyst.

In the manufacturing method according to the present embodiment, a catalyst is used. As the catalyst, an oxide containing at least one metal selected from the group consisting of metals of group 2 to group 13 in the periodic table, lanthanoids and actinoids may be used.

The catalyst may be an oxide containing a transition metal or may be an oxide containing a metal other than a transition metal, but it should preferably be an oxide containing a transition metal from the aspect of reducing solids in the product.

When the catalyst is an oxide containing a transition metal, the catalyst is more preferably a composite oxide containing a transition metal, more preferably a composite oxide containing zirconium, and still more preferably a composite oxide containing zirconium and tungsten. A specific example of a composite oxide containing zirconium and tungsten is zirconia tungstate or the like.

When the catalyst is an oxide containing a metal other than a transition metal, the catalyst is preferably an aluminum oxide or the like. A specific example of an aluminum oxide is α-alumina or the like.

Furthermore, the catalyst is preferably a catalyst that is supported on a carbon material or an aluminum oxide. The carbon material is not particularly limited, but examples include activated carbon, carbon black, graphite, carbon fibers, carbon nanotubes, carbon nanohorns, fullerene and the like. Additionally, the aluminum oxide is not particularly limited, and for example, α-alumina may be used.

The catalyst content is not particularly limited, and for example, it is preferable to use 0.1 to 10 wt% relative to the methanol or ethanol, or to use 1 to 100 g/mL/min in terms of the ratio (the so-called W/F value) between the catalyst weight and the reactant space velocity.

The microalgae containing the oil/fat used in the method for manufacturing the bio-liquid fuel in the present embodiment is not particularly limited, but examples include microalgae such as *Chlorella* (including the phylogenetically separate *Parachlorella*), *Senedesmus, Botryococcus, Stichococcus, Nannochloris, Desmodesmus* and *Nannochloropsis.* More specifically, examples include species of *Chlorella* such as *Chlorella kessleri, Chlorella vulgaris* and *Chlorella saccharophila; Parachlorella kessleri* (*Chlorella kessleri*), which is classified as a *Trebouxiophycea* by molecular phylogenetic analysis; *Senedesmus obliquus,* which belongs to the genus *Senedesmus; Stichococcus ampliformis,* which belongs to the genus *Stichococcus; Nannochloris bacillaris,* which belongs to the genus *Nannochloris; Desmodesmus subspicatus,* which belongs to the genus *Desmodesmus;* and *Nannochloropsis oculata,* which belongs to the genus *Nannochloropsis.* In addition thereto, examples include diatoms and *Trebouxia, Pseudochoricystis, Euglena, Haematococcus,* cyanobacteria such as *Spirulina* (*Arthrospira*), and further thereto, red algae such as *Galdieria* and green algae. In the manufacturing method in the present embodiment, it is also possible to use genetically modified cyanobacteria or microalgae. Additionally, it is possible to use oomycetes that do not perform photosynthesis, such as *Aurantiochytrium.*

The microalgae used in the present embodiment may be dried or may contain water. In other words, the microalgae may be reacted with methanol or ethanol as mentioned below after being fully dried, or may be reacted with methanol or ethanol in a state still containing a small amount of moisture after removing the water.

By performing the reaction without drying the microalgae, the energy and cost required for drying become unnecessary.

Although the method for bringing the microalgae containing an oil/fat into contact with the supercritical or subcritical methanol, or the supercritical or subcritical ethanol, is not particularly limited, it is possible to use a batch method or a flow method.

In the case of a batch method, the reaction may be performed by loading the microalgae, the methanol or ethanol, and the catalyst in a single container capable of withstanding high temperatures and high pressures, sealing the container, and in this state, heating and/or pressurizing the container to create conditions in which the methanol or ethanol in the container becomes supercritical or subcritical, and holding these conditions for a prescribed period of time.

Additionally, in the case of a flow method, the reaction may be performed by causing the microalgae and the methanol or ethanol to flow, in the presence of the above-mentioned catalyst, inside a pipe that is able to withstand high temperatures and high pressures, under conditions in which the methanol or ethanol enters a supercritical or subcritical state as described below.

The reaction container is not particularly limited, but it is possible to use, for example, one composed of stainless steel or the like from the aspect of paying appropriate heed to the pressure resistance.

The critical point of methanol occurs at 239.5 °C and 8.09 MPa. Supercritical methanol refers to methanol in a state in which the temperature and pressure are in a temperature or pressure range at least as high as the critical temperature and the critical pressure of methanol, so that there is no distinction between the gas and liquid states. Subcritical methanol refers to liquid methanol in which the temperature and pressure are in a temperature or pressure range slightly lower than the critical temperature and the critical pressure of methanol.

Additionally, the critical point of ethanol occurs at 240.8 °C and 6.14 MPa. Supercritical ethanol refers to ethanol in a state in which the temperature and pressure are in a temperature or pressure range at least as high as the critical temperature and the critical pressure of ethanol, so that there is no distinction between the gas and liquid states. Subcritical ethanol refers to liquid ethanol in which the temperature and pressure are in a temperature or pressure range slightly lower than the critical temperature and the critical pressure of ethanol.

In the present embodiment, it is preferable to make the microalgae react with the methanol or ethanol, in the presence of the catalyst, under conditions in which the methanol or ethanol enters a supercritical or subcritical state, at a temperature from ordinary temperature to 400 °C and a pressure from ordinary pressure to 50 MPa. In the present embodiment, cell wall destruction of the microalgae, oil extraction from the microalgae and esterification of the extracted oils can be efficiently performed by using supercritical or subcritical methanol or supercritical or subcritical ethanol. As the reaction conditions are changed to higher temperatures and higher pressures, the reaction rates of cell wall destruction, oil extraction and esterification can be made faster. For example, in the method of the present embodiment, the reaction temperature is preferably 200 to 400 °C, more preferably 250 to 400 °C.

In the present embodiment, reactions can be performed whether (1) anhydrous methanol or anhydrous ethanol is to be reacted with dried microalgae, (2) water-containing methanol or water-containing ethanol is to be reacted with dried microalgae, (3) anhydrous methanol or anhydrous ethanol is to be reacted with water-containing microalgae, or (4) water-containing methanol or water-containing ethanol is to be reacted with water-containing microalgae.

The proportional water content relative to the microalgae and the methanol or ethanol overall, whether in the case in which the microalgae contain water, the case in which the methanol or ethanol contains water, or the case in which both the microalgae and the methanol or ethanol contain water, is preferably 30 wt% or less, more preferably 25 wt% or less, and even more preferably 20 wt% or less, when the total weight of the methanol or ethanol and water is 100%.

For example, if the proportional water content is 20 wt% of the total weight of methanol and water, the critical point of the mixed solvent of methanol and water is 270.2 °C and 10.6 MPa, as calculated on the basis of the Lorentz-Berthelot rules.

The blending ratio between the microalgae and the methanol or ethanol may be appropriately set in accordance with the reaction scale or the like.

The method for heating the microalgae and the methanol or ethanol is not particularly limited, but examples include methods of heating by means of molten salt baths or electric furnaces.

The reaction time of the microalgae with the methanol or ethanol under the abovementioned reaction conditions is not particularly limited, and may be appropriately set in accordance with the reaction scale or the like.

According to the method for manufacturing the bio-liquid fuel in the present embodiment, an oxide containing at least one metal selected from the group consisting of metals of group 2 to group 13 in the periodic table, lanthanoids and actinoids is used as a catalyst. Therefore, cell destruction and triglyceride esterification are promoted, so that a step of destroying the cell walls of the microalgae, a step of converting triglycerides contained in the microalgae to fatty acid esters, and a step of extracting the generated fatty acid esters can be performed simultaneously and efficiently, in a single step.

Additionally, according to the method for manufacturing the bio-liquid fuel in the present embodiment, it is possible to extract oils other than triglycerides contained in the microalgae.

Additionally, according to the method for manufacturing the bio-liquid fuel in the present embodiment, it is possible to convert some of the other organic matter contained in the microalgae to oils and to extract the oils.

According to the method in the present embodiment, particularly in the case in which water is contained in the reaction system, there is an effect of reducing the solids in the resulting product in comparison to the case in which a catalyst is not used.

### [Examples]

The present invention will be explained in further detail by providing examples below. However, the present invention is not limited, in any way, by these examples.

### <Product Analysis>

The generated gases were analyzed by means of gas chromatography (GC) using the detectors indicated below in accordance with the type of gas:
[1] Quantification of CH₄, CO₂, H₂, CO and air: thermal conductivity detector (TCD)
[2] C2 or higher gas products: hydrogen flame ionization detector (FID)

Liquid products (oils) were analyzed with a GC-FID using a capillary column to quantify the fatty acid methyl esters (FAME).

Additionally, qualitative analysis and quantitative analysis of the oils were performed using GC-MS.

### [Example 1]

As a batch reaction container, a stainless steel pipe (SUS316, outer diameter 1/2 inch, thickness 2.1 mm, length 15 cm) provided with a high-pressure needle valve on one end, with a stainless steel tube (SUS316, outer diameter 1/8 inch) therebetween, was used. Approximately 0.56 g of *C*. *vulgaris* powder (manufactured by Chlorella Industry Co., Ltd.; average particle size 76 µm), 4.0 g of anhydrous methanol, and 0.028 g of α-alumina, as a catalyst, were loaded into the reaction container.

The above-mentioned batch reaction container was immersed in a molten salt bath that was pre-heated to 385 °C, and after 60 minutes elapsed, the reaction container was pulled up from the molten salt bath and rapidly cooled to room temperature by means of running water.

The generated gas was recovered by using a gas bag and the volume was measured by a water displacement method.

The solid product was recovered by using filter paper to filter the liquid product.

The liquid product was extracted by using methanol and hexane, and the oils were obtained by separating the hexane layer and performing vacuum distillation of the hexane.

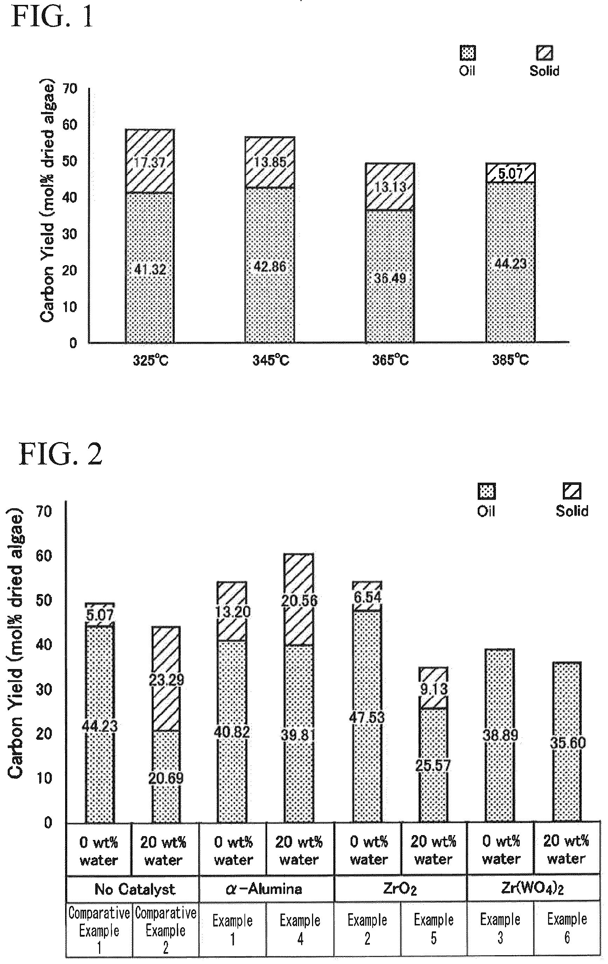

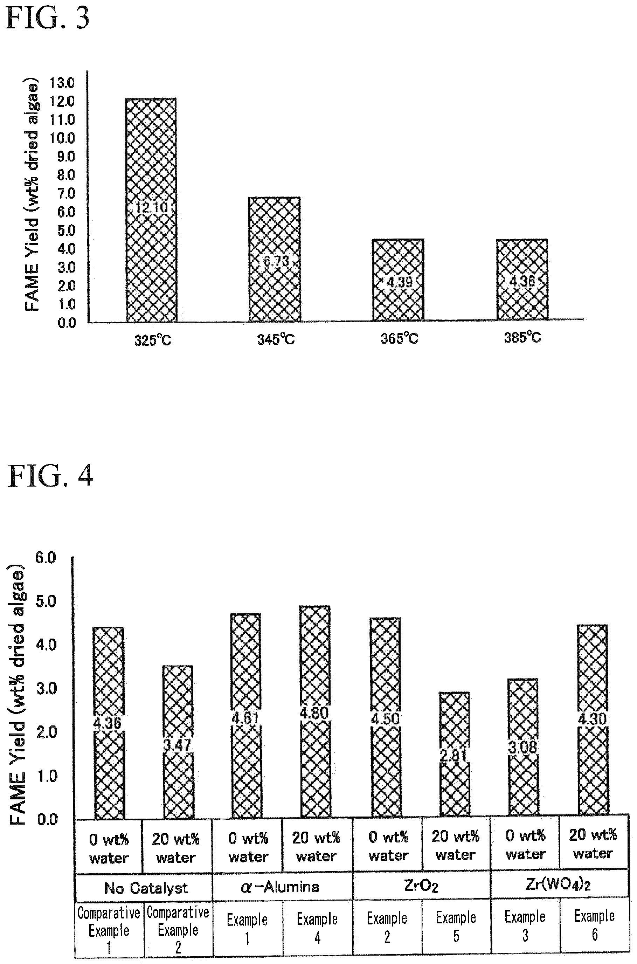

The results thereof are shown in Fig. 2 and Fig. 4.

### [Example 2]

A procedure similar to that of Example 1 was used, other than the fact that zirconium oxide was used as the catalyst.

The results thereof are shown in Fig. 2 and Fig. 4.

### [Example 3]

A procedure similar to that of Example 1 was used, other than the fact that zirconia tungstate was used as the catalyst.

The results thereof are shown in Fig. 2 and Fig. 4.

### [Example 4]

A procedure similar to that of Example 1 was used, other than the fact that α-alumina was used as the catalyst, and methanol containing 20 mass% of water was used instead of anhydrous methanol.

The results thereof are shown in Fig. 2 and Fig. 4.

### [Example 5]

A procedure similar to that of Example 4 was used, other than the fact that zirconium oxide was used as the catalyst.

The results thereof are shown in Fig. 2 and Fig. 4.

### [Example 6]

A procedure similar to that of Example 4 was used, other than the fact that zirconia tungstate was used as the catalyst.

The results thereof are shown in Fig. 2 and Fig. 4.

### [Comparative Example 1]

A procedure similar to that of Example 1 was used, other than the fact that a catalyst was not added, the batch reaction container was immersed in a molten salt bath that was pre-heated to the temperatures shown in Fig. 1, and after 60 minutes elapsed, the reaction container was pulled up from the molten salt bath and rapidly cooled to room temperature by means of running water.

The results thereof are shown in Fig. 1 to Fig. 4.

### [Comparative Example 2]

A procedure similar to that of Comparative Example 1 was used, other than the fact that methanol containing 20 mass% of water was used instead of anhydrous methanol, and the temperature of the molten salt bath was set to 385 °C.

The results thereof are shown in Fig. 2 and Fig. 4.

As shown in Fig. 2 and Fig. 4, Example 1 had a lower oil yield than Comparative Example 1 did, but had a higher fatty acid ester yield than Comparative Example 1 did. Example 3 had lower oil and fatty acid ester yields than Comparative Example 1 did, but the product did not contain solids.

In comparison to Comparative Example 2, in which a catalyst was not used, Examples 4 to 6, in which water was included in the reaction, had a high oil yield and fewer solids. Examples 4 and 6 had a higher fatty acid ester yield than Comparative Example 2 did. In Example 6, the product did not contain solids.

### [Industrial Applicability]

The present invention can provide a method for manufacturing a bio-liquid fuel, wherein cell wall destruction, oil extraction and esterification are simultaneously performed in a single step.

## Claims

1. A method for manufacturing a bio-liquid fuel wherein microalgae containing an oil/fat are brought into contact with a supercritical or subcritical methanol, or a supercritical or subcritical ethanol, in the presence of a catalyst that is an oxide containing at least one metal selected from the group consisting of metals of group 2 to group 13 in the periodic table, lanthanoids and actinoids.

2. The method for manufacturing a bio-liquid fuel as in claim 1, wherein the catalyst is an oxide containing a transition metal.

3. The method for manufacturing a bio-liquid fuel as in claim 1 or 2, wherein the catalyst is a catalyst supported on a carbon material or an aluminum oxide.

4. The method for manufacturing a bio-liquid fuel as in any one of claims 1 to 3, wherein the catalyst is a composite oxide containing a transition metal.

5. The method for manufacturing a bio-liquid fuel as in any one of claims 1 to 4, wherein the catalyst is a composite oxide containing zirconium.

6. The method for manufacturing a bio-liquid fuel as in any one of claims 1 to 5, wherein the catalyst is a composite oxide containing zirconium and tungsten.

7. The method for manufacturing a bio-liquid fuel as in any one of claims 1 to 6, wherein the catalyst is zirconia tungstate.

8. The method for manufacturing a bio-liquid fuel as in any one of claims 1 to 7, wherein at least one of the microalgae containing the oil/fat, the supercritical or subcritical methanol, and the supercritical or subcritical ethanol contains water.

9. The method for manufacturing a bio-liquid fuel as in any one of claims 1 to 8, wherein the microalgae containing the oil/fat are brought into contact with the supercritical or subcritical methanol, or the supercritical or subcritical ethanol, at a temperature from ordinary temperature to 400 °C and at a pressure from ordinary pressure to 50 MPa.

10. The method for manufacturing a bio-liquid fuel as in any one of claims 1 to 9, wherein the microalgae containing the oil/fat are brought into contact with the supercritical or subcritical methanol, or the supercritical or subcritical ethanol, at a temperature from 200 to 400 °C and at a pressure from ordinary pressure to 50 MPa.
